# EUROPEAN PATENT SPECIFICATION

(11) **EP 0 721 332 B1**
(45) Date of publication and mention of the grant of the patent: **31.07.2002**
(21) Application number: 94929077.9
(22) Date of filing: 29.09.1994
(51) Int. Cl.: A61K 9/16, A61K 9/72, B01J 2/00

(54) **PROCESS II**
VERFAHREN II
PROCEDE (II)

(30) Priority: 01.10.1993 SE 9303215; 22.12.1993 SE 9304270
(43) Date of publication of application: 17.07.1996
(73) Proprietor: AstraZeneca AB, 151 85 Södertälje (SE)
(72) Inventor: TROFAST, Eva, S-226 47 Lund (SE); OLSSON, Magnus, S-223 50 Lund (SE); AHLNECK, Claes, S-211 31 Malmö (SE)
(86) International application number: PCT/SE94/00897
(87) International publication number: WO 95/09616

(56) References cited:
- EP-A- 0 072 046
- EP-A- 0 291 201
- GB-A- 2 187 952
- US-A- 4 161 516
- US-A- 5 143 126

## Description

The invention relates to a method and an apparatus for making a powder consisting of particles having a particle size smaller than 10 µm free-flowing by forcing the particles to create agglomerates.

Powders consisting of very small particles are commonly used in the inhalation therapy where the size of the particles are of utmost importance. The diameter of particles which are to be inhaled must be less than 10 µm to ensure the adequate penetration of the particles into the bronchial area of the lungs.

Most finely divided powders, such as micronized powders, are light, dusty and fluffy and they often create problems during handling, processing and storing. For particles having a diameter less than 10 µm the van der Wahls forces are generally greater than the force of gravity and consequently the material is cohesive. The particles tend to adhere to each other forming non-defined agglomerates. Such powders have very poor free-flowing properties which often make handling and precise metering problematic.

One possible method of making these powders free-flowing or at least improve their flowing properties is to force, in a controlled manner, the primary particles to form larger particles, agglomerates. Non-defined agglomerates are formed at random when this finely divided material is handled, for instance during storage, conveying, sieving, sifting, mixing or grinding.

It is common knowledge that spherical agglomerates flow freely, packs easily and uniformly, have an ideal form for coatings and are therefore commonly used in drug formulations.

The flow of very cohesive powders can be improved by vibration-induced agglomeration. Depending on the type of powder, liquid (often water) or solid binders are added during the agglomeration, but it is also possible to agglomerate without binders.

The method of agglomeration is applicable in principle to all materials, including mixtures of various powders. Any powder, provided it contains a sufficient amount of fine particles having a size smaller than 10 µm, can be granulated or pelletized without a binder by systematic agitation or rolling of the particulate material.

The agglomerated powders consist of relatively large, more dense and compact spheres which exhibit the normal flow properties, but at the same time the spheres should have sufficient low internal coherence to break up into small primary particles of medicament of a therapeutically effective size during inhalation in an inhalation device.

The inhaled route of administration enables the dose to be delivered directly to the airways. By this type of administration it is possible to give a small dose and thereby minimizing unwanted side effects, which for example could occur if the substance is deposited in another part of the body, e.g. the gastrointestinal tract or the oro-laryngeal tract.

### Prior art

Methods of controlled agglomeration are known in the prior art. For example, Claussen and Petzow (Journal of Materials Technology, vol 4(3), 148-156 (1973)) have described a dry agglomeration method where no binders are intentionally added for preparation of spheres in the size range 0.1 - 3 mm by tumbling in a cylinder tilted at an angle to the horizontal axis of rotation. According to the authors a dry agglomeration process for small particles requires nuclei in order to start, but almost all powders consist of natural agglomerates that function as nuclei. They also conclude that agglomerates from common used devices such as a rotating drum or a granulating pan form a wide size distribution of the agglomerates that makes frequent sieving necessary. The product formed often exhibit bad sphericity and low density.

US-A-5 143 126 describes a vibratory conveyor for forming flowable agglomerates from previously poorly flowable fine-grained powder by using a method wherein the poorly flowable powder is subjected to a mechanical vibration step prior to transport and metering.

GB-A-1 569 611 describes a process for agglomeration of a drug into soft pellets. In this process moist is used as a binder to provide a doe which by extrusion is pressed through a sieve to create agglomerates.

GB-A-2 187 952 describes a method in which crystalline ibuprofen is compacted by kneading as it is conveyed by conveying screws through an extruder. The resulting agglomerates can also be passed through an extruder plate affixed to an end of the extruder.

EP-A-0 490 649 a laboratory process for obtaining soft pellets is described. In this process a sieve was used having an aperture size between 210 to 500 microns. A palett knife was used to extrude the powder through the sieve. The extrudate formed was placed in a glass jar which was placed on a set of rollers in order to further treat the extrudate.

### The invention

It is an object of the present invention to provide a method of controlled agglomeration of finely divided powdered medicament having a primary particle size smaller than 10 µm, preferably smaller than 5 µm, for example micronized powders, in which no binders are needed and in which the resulting agglomerates are of a uniform size having a structure which provides sufficient flowability for the transport and metering of such powders and which nevertheless have sufficient low internal coherence to break up, within an inhalation device, such as a dry powder inhalator, into particles of medicament having a therapeutically effective size, e.g. having a particle size smaller than 10 µm.

The method according to the invention provides a process for facilitating the technical handling and significantly increase the medical value of the substance. It has been found that this method produces agglomerates having excellent handling properties, which have sufficient strength to withstand packaging and storage, but which are sufficient soft so that they will break down into primary particles when they are expelled from the inhalator during inhalation therapy.

According to the invention there is provided a method for the manufacture of agglomerates, which comprises subjecting the finely divided particles of the medicament, which could be in admixture with any other ingredient desired to be incorporated into the agglomerates, to mechanical unit operations under certain conditions. More specifically there is provided a method of treatment of a finely divided powdered medicament having a particle size smaller than 10 µm and poor flowing properties to form, in a controlled manner, agglomerates or pellets which are free flowing and which are capable of breaking down to provide the finely divided medicament, comprising the steps of
a) agglomerating the powdered medicament by feeding the material to a sieve having a U-shaped form, mechanically causing the finely divided powdered medicament to pass through the apertures of the sieve by using a mechanically driven rotor device thereby obtaining agglomerates,
b) spheronizing the resulting agglomerates in order to provide agglomerates which are more spherical, more dense and more compact than the agglomerates obtained from the agglomeration process in the sieve, and
c) sizing the agglomerates to obtain an uniform size of the final product.

According to the invention there is also provided an apparatus for performing the method of treatment of a finely divided powdered medicament having a particle size smaller than 10 µm and poor flowing properties to form, in a controlled manner, agglomerates or pellets which are free flowing and which are capable of breaking down to provide the finely divided medicament, comprising a sieve having apertures through which the finely divided powder is mechanically caused to pass to obtain agglomerates, a spheronizing device and a sizing device for sizing the agglomerates to obtain a uniform size of the final product.

Further preferred steps of the method are clear from the appended dependent claims 2 - 13 and preferred embodiments of the apparatus are clear from the appended dependent claims 14 - 25.

There is also provided a use of the apparatus to carry out the method according to the invention.

It is also an object of the invention to provide a use of the agglomerates manufactured in accordance with the method in a breath actuated dry powder inhaler, such as Turbuhaler®.

### Brief description of the drawings

The method according to the present invention will now be described by way of example with reference to the appended drawings, wherein:
Fig. 1 shows a schematic view of a first embodiment of the apparatus and process according to the invention,
Fig. 2 is a sectional view along line A - A in fig. 1,
Fig. 3 shows a schematic view of an alternative of the apparatus and process shown in fig. 1,
Fig. 4 shows a schematic view of the apparatus according to the invention having a second embodiment of the agglomerating sieve,
Fig. 5 is a diagram showing size distribution of spheres as a function of different sizes of the apertures in the used sieve.

### Detailed description of the drawings

According to the invention the finely divided powdered medicament is supplied to a sieve 2, 208 through which apertures the powder is forced. Mechanical devices are used to press the powder through the apertures. During this treatment small, soft agglomerates or pellets are formed which are capable of breaking down to provide the finely divided medicament and which could be spheronized to obtain a more spherical, dense and stable form. The agglomerates resulting from the spheronizing process are harder than the agglomerates resulting from the agglomeration process but are still capable of breaking down to provide the finely divided medicament which, as already mentioned above, is of utmost importancy when the agglomerates are to be used in the inhalation therapy.

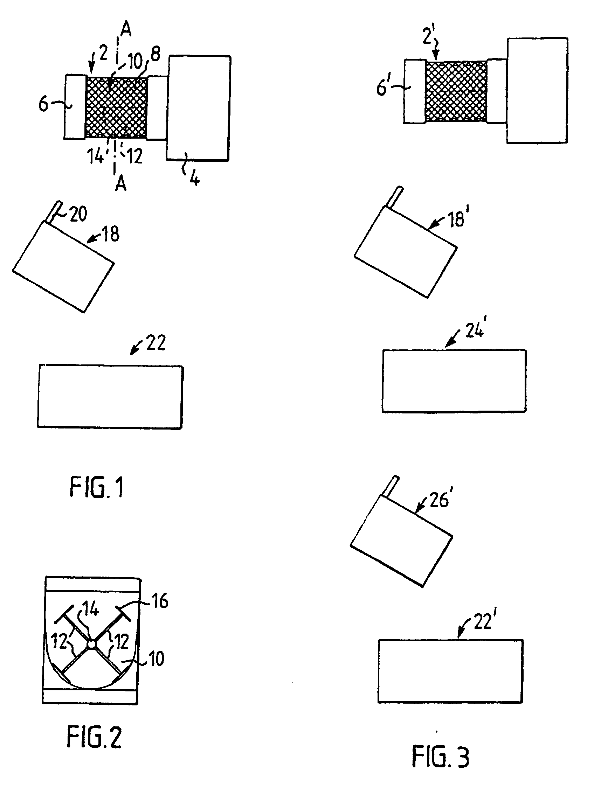

A first embodiment of the apparatus for carrying out the method according to the invention can be seen in fig. 1. The sieve 2 which is used for the agglomeration is hereby formed as a trough 6 having a substantially U-shaped form. The walls of the U-shaped trough are made of a net 8 of any rigid material such as metal, plastic or other suitable material.

The size of the resulting agglomerates is depending on the size of the apertures in the net 8. To obtain agglomerates which have a size and form which make them suitable for the following spheronization treatment, which is described in more details below, the size of the apertures should be between 0.2 - 2.0 mm, preferably between 0.3 - 1.0 mm.

Inside the U-shaped trough an oscillating and/or rotating device 10 is provided. This oscillating device 10 is preferably provided with at least one arm 12 mounted on a shaft 14 which is arranged along the longitudinal axis of the U-shaped trough 6 as shown in fig. 1 and 2. In the preferred embodiment the oscillating device 10 is provided with four arms 12 mounted perpendicular to each other. In the end of each arm a plate 16 is mounted in a right angle to the arm 12, see fig. 2. These plates will, due to the oscillating movement of the oscillating device 10, when oscillating and/or rotating, force the finely divided powder supplied to the U-shaped trough 6 through the apertures of the net 8 thereby forming the agglomerates.

The shaft 14 of the oscillating device 10 is mounted to a motor 4 or similar which is provided to produce and transmit the oscillating and/or rotating movement to the device.

The agglomerates obtained from the sieve after the agglomeration process have different sizes and are comparatively soft, they need to be further treated to obtain the desired characteristics. The agglomerates are therefore collected in a spheronizing device, preferably a rotating container, such as a pan or drum 18 which preferably is provided with one or more scrapers 20 (only shown schematically in the drawings). The container 18 is tilted and is rotating. The rotating movement of the container 18 will make the agglomerates rotate and tumble due to the tilting of the container. The scrapers will give the agglomerates a further rotation and shaping thereby improving the spheronization. During the rotation the agglomerates will obtain a stronger, more dense, compact and uniform form and a smoother outer surface. These improvements in form, hardness and density achieved in the rotating container 18 will further improve the flowability and the resistance against breaking during handling and storage. The speed of the container determines the characteristics of the agglomerates after this spheronization. Tests have shown that the optimal periphery speed of the container is between 0.2 - 2.0, preferably between 0.4 - 1.0 m/s. The spheronization time is preferably between 1 - 20 min. Tests have shown that after 3 - 10 min the agglomerates often have obtained the required optimal size, capability of breaking down to provide the finely divided medicament and density for their future use.

Tests have shown that the most optimate tilting angle of the container 18 is between 10°- 80° from the vertical, preferably between 30°- 60° as an angle chosen herebetween gives the best densifying and growing effect to the agglomerates.

The granulating container is made of a material which is inert and do not contaminate the powder, such as for example metal, plastic or any other suitable material. In order to avoid electrostatic forces to build up during the spheronization process the container could be grounded.

After the spheronization in the tilted container 18 the agglomerates are supplied to a sizing device, preferably a sieve 22 having an aperture size which is between 0.2 - 2.0 mm, preferably between 0.3 - 1.0 mm. This final sieving is used in order to obtain a uniform size of the agglomerates. The requirements for the use of this operation is strongly depending on the type of inhalation device to be used.

The requirements of uniform size and proper density is higher if the agglomerates are to be used in a dry powder inhaler. During inhalation it is of utmost importance that agglomerates are broken up into a high amount of primary particles having a particle size smaller than 10 µm.

To utilize the process in the most efficient and economic manner and to minimize the amount of agglomerates which are too big and therefore have to be recycled into the process the method could comprise further steps of sieving and spheronization. The final agglomerates will also be more uniform which will improve the break down of the agglomerates into primary particles during inhalation. A further step of sieving is incorporated hereby into the process after the spheronization and a second spheronization is incorporated after this extra sieving. An apparatus according to this embodiment of the invention is shown in fig 3. The finely divided powdered medicament is agglomerated in the substantially U-shaped trough 6' and the resulting agglomerates are supplied to the granulating container 18'. After the spheronization the agglomerates are supplied to a sieve 24' to obtain a more uniform size. After this sieving the agglomerates are spheronized a second time in a second granulating container 26'. This second granulating container 26' is of the same type as the first container 18' and the periphery speed and the spheronization time as defined above for the first step of spheronization. After this second spheronization the agglomerates are sifted through the final sieve 22' to obtain a uniform size of the final product. The sifting is necessary as in some case the agglomerates might grow too much during the spheronization and therefore the final product could contain agglomerates having a size being bigger than the required size, e.g. 0.2 - 2 mm, preferably 0.3 - 1 mm.

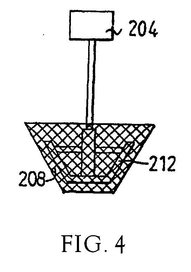

A second embodiment of the apparatus for carrying out the method according to the invention can be seen in fig. 4. The net 208 of the sieve is formed as a truncated cone and is provided with a scraper device 212. The scraper device could have any form but is preferably formed a horisontal element mounted on a shaft. At least two arms extends outwardly form the horisontal element in the angle of the net 208, e.g. the truncated cone. The shaft is connected to a motor 204 which gives the scraper device 212 a rotating movement. During the agglomeration process the rotation of the scrapers will force the finely divided particles of the powder to pass through the apertures in the net 208 thereby forming well defined agglomerates having the required characteristics.

After agglomeration the agglomerates are treated as described in relation to embodiment one of the present invention.

The agglomeration process according to the invention will now be described by experiments which are intended to illustrate the invention as described in the appended claims.

The agglomerates obtained from the process according to the invention are to be used in a dry-powder inhalator, preferably in a dry-powder breath-actuated inhalator. The hardness of the agglomerates are therefore of utmost importancy. The required hardness of agglomerates which are capable of breaking down into primary particles during inhalation has been measured by a MHT-4 Microhardness tester, (A.Paar, Austria) and has been found to vary between 0.5 to 20 mN for agglomerates having good deagglomeration properties and which break down into the required primary particles in an inhalator during inhalation. With values above 20 mN the deagglomeration of the agglomerates will be less and above 100 mN very little deagglomeration of the agglomerates will occur.

### Example 1

Properties of agglomerates of three different powders have been determined and is to be seen in the table below. The powder consisted of finely divided particles which was passed through the steps of the method according to the invention:

| SUBSTANCE | MASS MEDIUM DIAMETER (µm) | SURFACE AREA (m²/g) | BULKDENSITY (g/ml) |
|---|---|---|---|
| Terbutaline | 1.7 | 9 | 0.25 |
| Budesonide | 2.0 | 6 | 0.24 |
| Lactose | 3.0 | 6 | 0.32 |

Typically the bulk density for agglomerated powders consisting of finely divided particles can be determined to vary between 0.2 mg to 0.4 g/ml for particles having a particle size of less than 10 µm. The surface area varies between substances but there is no difference between micronised and micronised and agglomerated (and spheronised) powder. The area is between 2 - 20 m²/g, preferably 3 - 12 m²/g.

### Example 2

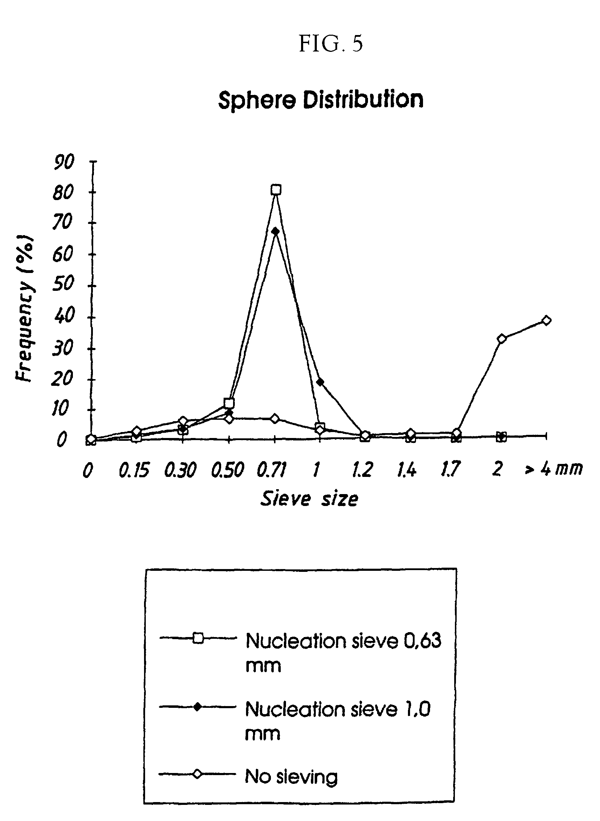

Micronized (mass medium diameter (MMD)) 3.2 µm lactose was slowly added on the oscillating device consisting of a U-shaped sieve net mounted on an Erweka AR 400. By the action of the bars of the oscillating device the lactose was pushed through the sieve net. The mesh size of the sieve net used was in one experiment 0.63 mm and in another experiment 1.0 mm. The oscillating frequency was in each case 90 turns/min. The agglomerates formed were collected and added to a stainless granulator (Eirisch type, 240 mm diameter), fixed at an angle of about 45° and equipped with a scraper, for growth of the spheres to occur. Tumbling of the agglomerates was performed at 50 rpm for 8 minutes. The spheres were collected and analyzed for size distribution in a Retsch sieve with a mesh size up to 2 mm. For comparison micronized lactose was spheronized without prior treatment in the oscillating sieve. The result is shown in the diagram in Fig. 5.

### Example 3

Agglomeration starts with particle-particle contact and adhesion (nucleation). These bodies act as nuclei for further growth of the agglomerates. The agglomeration step according to the present invention will create small spheres of relatively uniform size in the tumbling process, while direct tumbling of the fine cohesive powder will give large spheres with a wide size distribution. The difference in sphere size is thus due to different growth occuring during the tumbling. Since sieving through an oscillating device with a small sieve mesh produced nuclei of controlled size, less unagglomerated fine particles were left to increase the size of the agglomerates. The existence of a lot of non-agglomerated fine particles will give an uncontrolled sphere growth during tumbling and to larger variations in size distributions and a larger average sphere diameter and average sphere volume. This is shown in the diagram below, where the average sphere diameter (msd) and weight average sphere volume (msv) for spheres obtained after eight minutes of growth in a stainless granulator has been calculated as well as the relative standard deviation (RSD).

| | MSD | RSD | MSV | RSD |
|---|---|---|---|---|
| Nucleation step | mm | % | mm³ | % |
| Sieve 0.63 | 0.813 | 1.4 | 0.335 | 3 |
| Sieve 1.0 | 0.851 | 7.4 | 0.433 | 9.8 |
| No sieve | 3.18 | 13.3 | 73.2 | 92.1 |

The experiments clearly show the narrow distribution of the sphere sizes obtained in a U-shaped sieve compared with a direct spheronization procedure of the primary finely divided powder. A more uniform size of the spheres with the smaller size aperture, preferably a size of 0.3 to 1.2 is therefore recommended. Large agglomerates/spheres may eventually break up and thereby giving a wide range of agglomerate/sphere size distribution, which gives less accurate doses.

As a consequence the described process according to the present invention gives an agglomerated powder with acceptable batch to batch variations of the final product. Small variations is of utmost importance for accuracy of a dose from an inhalation device.

### Possible modifications

The method according to the invention could of course be modified within the scope of the appended claims as well as the apparatus.

Thus the shape of the sieve or the extrusion device where the controlled agglomeration takes place could be varied as well as the size of the apertures. This size must be chosen in regard to the characteristics of the finely divided powdered medicament to be agglomerated. The apertures of the sieve could for example have different shape, e.g. being round or having any other preferred shape.

The oscillating and/or rotating device or mechanical scraper device which forces the powder through the apertures of the net could have any suitable form. For example in the first embodiment with the U-formed trough the device could be formed as a tape being provided with wings arranged on the tape perpendicular to the net. Other forms are also possible within the scope of the claims.

It is also possible to modify the size, shape, speed and tilting angle of the granulating container thereby changing the size of the final agglomerates.

The spheronization could also be done in a so called marumerizer which is a commercially available apparatus for spheronization or granulation. The spheronization could also be done in any other suitable way using a rotation symmetrical receptacle or container, which could be rotated, such as any container being cylindrical or barrel formed.

## Claims

1. Method of treatment of a finely divided powdered medicament having a particle size smaller than 10 µm and poor flowing properties to form, in a controlled manner, agglomerates or pellets which are free flowing and which are capable of breaking down to provide the finely divided medicament, comprising the steps of
a) agglomerating the powdered medicament by feeding the material to a sieve having a U-shaped form, mechanically causing the finely divided powdered medicament to pass through the apertures of the sieve by using a mechanically driven rotor device thereby obtaining agglomerates,
b) spheronising the resulting agglomerates in order to provide agglomerates which are more spherical, more dense and more compact than the agglomerates obtained from the agglomeration process in the sieve, and
c) sizing the agglomerates to obtain an uniform size of the final product.

2. Method according to claim 1, **characterised in that** a tilted granulating container is used to spheronise the agglomerates resulting from the agglomeration.

3. Method according to claim 2, **characterised in that** said granulating container having one or more scrapers.

4. Method according to claim 1, 2 or 3, **characterised in that** a sieve is used for the sizing of the resulting agglomerates.

5. Method according to any of claim 1, **characterised in that** the particle size of the finely divided powdered medicament is smaller than 10µm and **in that** the size of the agglomerates after the agglomeration process is less than or equal to 2 mm.

6. Method according to claim 1, **characterised in that** the apertures of the sieve in the form of a U-shaped trough have a size between 0.2 - 2.0 mm.

7. Method according to claim 6, **characterised in that** the apertures of the sieve have a size between 0.3 - 1.0 mm.

8. Method according to any one of claims 1 to 7, **characterised in that** the method comprises further steps of sizing and spheronising after the initial steps of agglomeration and spheronisation.

9. Method according to claim 8, **characterised in that** for the further sizing a further sieve is used and for the further spheronising a further granulating container is used.

10. Method according to claim 9, **characterised in that** one or more scrapers is provided in said further granulating container.

11. Method according to claim 9 or 10, **characterised in that** the apertures of the further sieve has a size between 0.2 - 2.0 mm.

12. Method according to claim 11, **characterised in that** the apertures of the further sieve has a size between 0.3 to 1.0 mm.

13. Method according to any of the preceding claims, **characterised in that** the periphery speed of the granulating container is 0.4 - 1.0 m/s.

14. Apparatus for forming a powdered medicament having a particle size smaller than 10 µm and poor flowing properties into agglomerates or pellets which are free flowing and which are capable of breaking down to provide the finely divided medicament, comprising:
a sieve in the form of a U-shaped trough, having apertures through which the finely divided powdered medicament is mechanically caused to pass using a mechanically driven rotor device to obtain agglomerates;
a spheronising device for spheronising the resulting agglomerates;
and a sizing device for sizing the agglomerates to obtain a uniform size of the final product.

15. Apparatus according to claim 14, **characterised in that** the spheronising device is a tilted granulating container.

16. Apparatus according to claim 15, **characterised in that** one or more scrapers are provided in said granulating container.

17. Apparatus according to claim 14, 15 or 16, **characterised in that** the sizing device is a sieve.

18. Apparatus according to any one of claims 14 to 17, **characterised in that** the U-shaped trough is provided with a oscillating and/or rotating device mounted inside the U-shaped trough.

19. Apparatus according to claim 18, **characterised in that** the oscillating and/or rotating device comprises a shaft provided along the longitudinal axis of the U-shaped trough and having at least one arm extending perpendicular to the shaft, the arm being provided with a plate-like extrusion arranged perpendicular to the arm and in contact with the surface of the net.

20. Apparatus according to any one of claims 14 to 19, **characterised in that** it comprises a further sieve for sieving the agglomerates and a further tilted granulating container.

21. Apparatus according to claim 20, **characterised in that** one or more scraper are provided in said further granulating container.

22. Apparatus according to any one of claims 14 to 21, **characterised in that** the apertures of the sieves have a size between 0.2 to 2.0 mm.

23. Apparatus according to claim 22, **characterised in that** the apertures of the sieves have a size between 0.3 to 1.0 mm.

24. Apparatus according to any one of claims 14 to 23, **characterised in that** the periphery speed of the granulating container is 0.4 - 1.0m/s.

25. Apparatus according to claim 24, **characterised in that** the spheronisation time is 1 to 20 min.

26. Use of an apparatus according to any of claims 14 to 25 to carry out a method according to any of claims 1 to 13.

27. Agglomerated powders manufactured in accordance with a method as described in claims 1 to 13 by using an apparatus as described in claims 14 to 25 for use in a breath-actuated dry powder inhaler.

28. Agglomerated powders according to claim 27 consisting of finely divided powders having a particle size smaller than 10 µm.

29. Agglomerated powders according to claim 28 having a surface area of between 2 and 20 m²/g.

30. Agglomerated powders according to claim 29 having a surface area of between 3 and 12 m²/g.

31. Agglomerated powders according to claim 28 or 29 having a bulk density of between 0.2 and 0.4 g/ml.

## Patentansprüche

1. Verfahren zur Behandlung eines fein verteilten pulverförmigen Medikaments mit einer Teilchengröße von weniger als 10 µm und schlechten Fließeigenschaften, um gesteuert Agglomerate oder Pellets zu bilden, die rieselfähig sind und zur Bereitstellung des fein verteilten Medikaments zerfallen können, bei dem man
a) das pulverförmige Medikament agglomeriert, indem man das Material einem U-förmigen Sieb zuführt und unter Einsatz einer mechanisch angetriebenen Rotorvorrichtung mechanisch dafür sorgt, dass das fein verteilte pulverförmige Medikament durch die Öffnungen des Siebs geht, wodurch Agglomerate erhalten werden,
b) zur Bereitstellung von Agglomeraten, die kugelförmiger, dichter und kompakter als die durch das Agglomerationsverfahren im Sieb erhaltenen Agglomerate sind, die sich ergebenden Agglomerate sphäronisiert und
c) zum Erhalt einer gleichmäßigen Größe des Endprodukts die Agglomerate sortiert.

2. Verfahren nach Anspruch 1, **dadurch gekennzeichnet, dass** ein Kippgranulierbehälter verwendet wird, um die sich aus der Agglomeration ergebenden Agglomerate zu sphäronisieren.

3. Verfahren nach Anspruch 2, **dadurch gekennzeichnet, dass** der Granulierbehälter einen oder mehrere Schaber aufweist.

4. Verfahren nach Anspruch 1, 2 oder 3, **dadurch gekennzeichnet, dass** zur Sortierung der erhaltenen Agglomerate ein Sieb verwendet wird.

5. Verfahren nach einem von Anspruch 1, **dadurch gekennzeichnet, dass** die Teilchengröße des fein verteilten pulverförmigen Medikaments weniger als 10 µm beträgt und dass die Größe der Agglomerate nach dem Agglomerationsverfahren kleiner gleich 2 mm ist.

6. Verfahren nach Anspruch 1, **dadurch gekennzeichnet, dass** die Öffnungen des Siebs in Gestalt eines U-förmigen Trogs zwischen 0,2 und 2,0 mm groß sind.

7. Verfahren nach Anspruch 6, **dadurch gekennzeichnet, dass** die Öffnungen des Siebs zwischen 0,3 und 1,0 mm groß sind.

8. Verfahren nach einem der Ansprüche 1 bis 7, **dadurch gekennzeichnet, dass** es nach den anfänglichen Agglomerierungs- und Sphäronisierungsschritten weitere Sortier- und Sphäronisierungsschritte umfasst.

9. Verfahren nach Anspruch 8, **dadurch gekennzeichnet, dass** für das weitere Sortieren ein weiteres Sieb und für das weitere Sphäronisieren ein weiterer Granulierbehälter verwendet wird.

10. Verfahren nach Anspruch 9, **dadurch gekennzeichnet, dass** in dem weiteren Granulierbehälter ein oder mehrere Schaber bereitgestellt sind.

11. Verfahren nach Anspruch 9 oder 10, **dadurch gekennzeichnet, dass** die Öffnungen des weiteren Siebs zwischen 0,2 und 2,0 mm groß sind.

12. Verfahren nach Anspruch 11, **dadurch gekennzeichnet, dass** die Öffnungen des weiteren Siebs zwischen 0,3 und 1,0 mm groß sind.

13. Verfahren nach einem der vorhergehenden Ansprüche, **dadurch gekennzeichnet, dass** die Umfangsgeschwindigkeit des Granulierbehälters 0,4 - 1,0 m/s beträgt.

14. Vorrichtung zur Bildung von Agglomeraten oder Pellets, die rieselfähig sind und zur Bereitstellung des fein verteilten Medikaments zerfallen können, aus einem pulverförmigen Medikament mit einer Teilchengröße von weniger als 10 µm und schlechten Fließeigenschaften, mit Folgendem:
einem Sieb in Gestalt eines U-förmigen Trogs mit Öffnungen, wobei unter Einsatz einer mechanisch angetriebenen Rotorvorrichtung mechanisch dafür gesorgt wird, dass das fein verteilte pulverförmige Medikament durch diese Öffnungen geht, wodurch Agglomerate erhalten werden,
einer Sphäronisiervorrichtung zum Sphäronisieren der erhaltenen Agglomerate
und einer Sortiervorrichtung zum Sortieren der Agglomerate, um eine einheitliche Größe des Endprodukts zu erhalten.

15. Vorrichtung nach Anspruch 14, **dadurch gekennzeichnet, dass** es sich bei der Sphäronisiervorrichtung um einen Kippgranulierbehälter handelt.

16. Vorrichtung nach Anspruch 15, **dadurch gekennzeichnet, dass** in dem Granulierbehälter ein oder mehrere Schaber vorgesehen sind.

17. Vorrichtung nach Anspruch 14, 15 oder 16, **dadurch gekennzeichnet, dass** es sich bei der Sortiervorrichtung um ein Sieb handelt.

18. Vorrichtung nach einem der Ansprüche 14 bis 17, **dadurch gekennzeichnet, dass** der U-förmige Trog mit einer darin angeordneten schwingenden und/oder sich drehenden Vorrichtung versehen ist.

19. Vorrichtung nach Anspruch 18, **dadurch gekennzeichnet, dass** die schwingende und/oder sich drehende Vorrichtung eine Welle umfasst, die entlang der Längsachse des U-förmigen Trogs vorgesehen ist und mindestens einen Arm aufweist, der sich senkrecht zur Welle erstreckt und mit einer plattenartigen Anformung versehen ist, die senkrecht zum Arm angeordnet ist und mit der Oberfläche des Netzes in Kontakt steht.

20. Vorrichtung nach einem der Ansprüche 14 bis 19, **dadurch gekennzeichnet, dass** sie ein weiteres Sieb zum Sieben der Agglomerate und einen weiteren Kippgranulierbehälter umfasst.

21. Vorrichtung nach Anspruch 20, **dadurch gekennzeichnet, dass** in dem weiteren Granulierbehälter ein oder mehrere Schaber vorgesehen sind.

22. Vorrichtung nach einem der Ansprüche 14 bis 21, **dadurch gekennzeichnet, dass** die Öffnungen der Siebe zwischen 0,2 und 2,0 mm groß sind.

23. Vorrichtung nach Anspruch 22, **dadurch gekennzeichnet, dass** die Öffnungen der Siebe zwischen 0,3 und 1,0 mm groß sind.

24. Vorrichtung nach einem der Ansprüche 14 bis 23, **dadurch gekennzeichnet, dass** die Umfangsgeschwindigkeit des Granulierbehälters 0,4 - 1,0 m/s beträgt.

25. Vorrichtung nach Anspruch 24, **dadurch gekennzeichnet, dass** die Sphäronisierungszeit 1 bis 20 min beträgt.

26. Verwendung einer Vorrichtung nach einem der Ansprüche 14 bis 25 zur Durchführung eines Verfahrens nach einem der Ansprüche 1 bis 13.

27. Agglomerierte Pulver, die gemäß einem Verfahren nach Ansprüchen 1 bis 13 unter Verwendung einer Vorrichtung nach Ansprüchen 14 bis 25 für die Verwendung in einem atemzugaktivierten Trockenpulverinhalator hergestellt wurden.

28. Agglomerierte Pulver nach Anspruch 27 aus fein verteilten Pulvern mit einer Teilchengröße von weniger als 10 µm.

29. Agglomerierte Pulver nach Anspruch 28 mit einer Oberfläche zwischen 2 und 20 m²/g.

30. Agglomerierte Pulver nach Anspruch 29 mit einer Oberfläche zwischen 3 und 12 m²/g.

31. Agglomerierte Pulver nach Anspruch 28 oder 29 mit einer Schüttdichte zwischen 0,2 und 0,4 g/ml.

## Revendications

1. Procédé de traitement d'un médicament en poudre finement divisé ayant une taille de particule inférieure à 10 µm et de médiocres propriétés d'écoulement pour former, d'une manière contrôlée, des agglomérats ou des granulés à écoulement facile capables de se désagréger pour fournir le médicament finement divisé, comprenant les étapes consistant à
a) agglomérer le médicament en poudre en chargeant le matériau dans un crible ayant une forme en U, en faisant passer mécaniquement le médicament en poudre finement divisé à travers les ouvertures du crible en utilisant un dispositif à rotor entraîné mécaniquement pour ainsi obtenir des agglomérats,
b) sphéroniser les agglomérats résultants afin de fournir des agglomérats qui sont plus sphériques, plus denses et plus compacts que les agglomérats obtenus à partir de l'opération d'agglomération dans le crible, et
c) calibrer les agglomérats pour obtenir une taille uniforme du produit final.

2. Procédé selon la revendication 1, **caractérisé en ce qu'**un récipient de granulation incliné est utilisé pour sphéroniser les agglomérats résultant de l'agglomération.

3. Procédé selon la revendication 2, **caractérisé en ce que** ledit récipient de granulation a un ou plusieurs racleurs.

4. Procédé selon la revendication 1, 2 ou 3, **caractérisé en ce qu'**un crible est utilisé pour le calibrage des agglomérats résultants.

5. Procédé selon l'une quelconque des revendications 1, **caractérisé en ce que** la taille de particule du médicament en poudre finement divisé est inférieure à 10 µm et **en ce que** la taille des agglomérats après l'opération d'agglomération est inférieure ou égale à 2 mm.

6. Procédé selon la revendication 1, **caractérisé en ce que** les ouvertures du crible en forme d'auge en U ont une taille comprise entre 0,2 et 2,0 mm.

7. Procédé selon la revendication 6, **caractérisé en ce que** les ouvertures du crible ont une taille comprise entre 0,3 et 1,0 mm.

8. Procédé selon l'une quelconque des revendications 1 à 7, **caractérisé en ce que** le procédé comprend les étapes supplémentaires consistant à calibrer et à sphéroniser après les étapes initiales d'agglomération et de sphéronisation.

9. Procédé selon la revendication 8, **caractérisé en ce que** l'on utilise un autre crible pour le calibrage supplémentaire et un autre récipient de granulation pour la sphéronisation supplémentaire.

10. Procédé selon la revendication 9, **caractérisé en ce qu'**un ou plusieurs racleurs sont prévus dans ledit récipient de granulation supplémentaire.

11. Procédé selon la revendication 9 ou 10, **caractérisé en ce que** les ouvertures du crible supplémentaire ont une taille comprise entre 0,2 et 2,0 mm.

12. Procédé selon la revendication 11, **caractérisé en ce que** les ouvertures du crible supplémentaire ont une taille comprise entre 0,3 et 1,0 mm.

13. Procédé selon l'une quelconque des revendications précédentes, **caractérisé en ce que** la vitesse périphérique du récipient de granulation est de 0,4 à 1,0 m/s.

14. Appareil pour former un médicament en poudre ayant une taille de particule inférieure à 10 µm et de médiocres propriétés d'écoulement en agglomérats ou granulés à écoulement facile capables de se désagréger pour fournir le médicament finement divisé, comprenant :
un crible en forme d'auge en U, ayant des ouvertures à travers lesquelles on fait passer mécaniquement le médicament en poudre finement divisé à l'aide d'un dispositif à rotor entraîné mécaniquement pour obtenir des agglomérats ;
un dispositif de sphéronisation pour sphéroniser les agglomérats résultants ;
et un dispositif de calibrage pour calibrer les agglomérats pour obtenir une taille uniforme du produit final.

15. Appareil selon la revendication 14, **caractérisé en ce que** le dispositif de sphéronisation est un récipient de granulation incliné.

16. Appareil selon la revendication 15, **caractérisé en ce qu'**un ou plusieurs racleurs sont prévus dans ledit récipient de granulation.

17. Appareil selon la revendication 14, 15 ou 16, **caractérisé en ce que** le dispositif de calibrage est un crible.

18. Appareil selon l'une quelconque des revendications 14 à 17, **caractérisé en ce que** l'auge en U est pourvue d'un dispositif oscillant et/ou rotatif monté à l'intérieur de l'auge en U.

19. Appareil selon la revendication 18, **caractérisé en ce que** le dispositif oscillant et/ou rotatif comprend un arbre prévu le long de l'axe longitudinal de l'auge en U et dont au moins un bras s'étend perpendiculairement à l'arbre, le bras étant pourvu d'une extrusion de type plaque disposée perpendiculairement au bras et en contact avec la surface du filet.

20. Appareil selon l'une quelconque des revendications 14 à 19, **caractérisé en ce qu'**il comprend un crible supplémentaire pour cribler les agglomérats et un récipient de granulation incliné supplémentaire.

21. Appareil selon la revendication 20, **caractérisé en ce qu'**un ou plusieurs racleurs sont prévus dans ledit récipient de granulation supplémentaire.

22. Appareil selon l'une quelconque des revendications 14 à 21, **caractérisé en ce que** les ouvertures des cribles ont une taille comprise entre 0,2 et 2,0 mm.

23. Appareil selon la revendication 22, **caractérisé en ce que** les ouvertures des cribles ont une taille comprise entre 0,3 et 1,0 mm.

24. Appareil selon l'une quelconque des revendications 14 à 23, **caractérisé en ce que** la vitesse périphérique du récipient de granulation est de 0,4 à 1,0 m/s.

25. Appareil selon la revendication 24, **caractérisé en ce que** la durée de sphéronisation est de 1 à 20 minutes.

26. Utilisation d'un appareil selon l'une quelconque des revendications 14 à 25, pour mettre en oeuvre un procédé selon l'une quelconque des revendications 1 à 13.

27. Poudres agglomérées fabriquées conformément à un procédé tel que décrit dans les revendications 1 à 13 en utilisant un appareil tel que décrit dans les revendications 14 à 25, pour l'utilisation dans un inhalateur de poudre sèche actionné par la respiration.

28. Poudres agglomérées selon la revendication 27, consistant en des poudres finement divisées ayant une taille de particule inférieure à 10 µm.

29. Poudres agglomérées selon la revendication 28, ayant une surface massique comprise entre 2 et 20 m²/g.

30. Poudres agglomérées selon la revendication 29, ayant une surface massique comprise entre 3 et 12 m²/g.

31. Poudres agglomérées selon la revendication 28 ou 29, ayant une densité en vrac comprise entre 0,2 et 0,4 g/ml.
